# EUROPÄISCHE PATENTANMELDUNG

(11) **EP 2 515 154 A2**
(43) Veröffentlichungstag der Anmeldung: **24.10.2012**
(21) Anmeldenummer: 12002730.5
(22) Anmeldetag: 19.04.2012
(51) Int. Cl.: G02B 23/24, A61B 1/07, A61B 1/06, A61B 1/00

(54) **Lichtleiteinrichtung für ein Endoskop zum Leiten von Beleuchtungslicht**

(30) Priorität: 21.04.2011 DE 102011007880
(71) Anmelder: Karl Storz GmbH & Co. KG, 78532 Tuttlingen (DE)
(72) Erfinder: Lei, Fang, 78532 Tuttlingen (DE); Weiger, Ulrich, 78532 Tuttlingen (DE); Bürk, André, 78532 Tuttlingen (DE)

(57) **Zusammenfassung**

Eine Lichtleiteinrichtung (20) für ein Endoskop, zum Leiten von Beleuchtungslicht zum distalen Ende (18) des Endoskops weist einen gekrümmten starren Abschnitt (272, 273) mit einer vorbestimmten räumlichen Konfiguration auf, wobei der gekrümmte starre Abschnitt (272, 273) zur Anordnung an einem distalen Ende eines Endoskops vorgesehen ist, wobei der gekrümmte starre Abschnitt (272, 273) seine starre Eigenschaft zumindest entweder bereits vor einem Einsetzen der Lichtleiteinrichtung (20) in ein Endoskop oder vor Ausbildung einer unmittelbaren oder mittelbaren mechanischen Verbindung der Lichtleiteinrichtung (20) mit einem Innenschaft (16) für ein Endoskop oder vor Ausbildung einer unmittelbaren oder mittelbaren mechanischen Verbindung der Lichtleiteinrichtung (20) mit einem Außenschaft (17) für ein Endoskop aufweist.

## Beschreibung

Die vorliegende Erfindung bezieht sich auf eine Lichtleiteinrichtung für ein Endoskop zum Leiten von Beleuchtungslicht zum distalen Ende des Endoskops, auf ein Endoskop mit einer Lichtleiteinrichtung sowie auf Verfahren zum Herstellen einer Lichtleiteinrichtung und zum Herstellen eines Endoskops.

In der medizinischen und technischen Endoskopie ist in der Regel eine Beleuchtung des betrachteten Objekts erforderlich. Zur Erzeugung von Beleuchtungslicht mit einem hohen Lichtstrom und erwünschten spektralen Eigenschaften, insbesondere einer guten Farbwiedergabe, werden oft separate oder in das proximale Ende des Endoskops integrierte Lichtquelleneinrichtungen verwendet. Vom proximalen Ende zum distalen Ende des Endoskops wird das Beleuchtungslicht mittels eines oder mehrerer Bündel von Lichtleitfasern übertragen.

Bei Konstruktion und Fertigung von Endoskopen ist zur präzisen Anordnung der Lichtleitfasern, insbesondere ihrer distalen Enden, ein hoher Aufwand erforderlich. Es gibt eine deutliche Tendenz, die Schäfte von Endoskopen immer dünner auszuführen. Der für die Lichtleitfasern zur Verfügung stehende Bauraum wird dabei immer kleiner. Dadurch steigt der konstruktive Aufwand, der erforderlich ist, um die notwenigen Mindest-Krümmungsradien für die Lichtleitfasern einzuhalten. Vor allem steigen der Aufwand bei der Fertigung und der erzeugte Ausschuss, da immer weniger Lichtleitfasern immer stärker gekrümmt werden.

Eine Aufgabe der vorliegenden Erfindung besteht darin, eine verbesserte Lichtleiteinrichtung zum Leiten von Beleuchtungslicht zu einem distalen Ende eines Endoskops, ein verbessertes Endoskop und verbesserte Verfahren zum Herstellen einer Lichtleiteinrichtung und eines Endoskops zu schaffen.

Diese Aufgabe wird durch die Gegenstände der unabhängigen Ansprüche gelöst.

Weiterbildungen sind in den abhängigen Ansprüchen angegeben.

Ausfiihrungsformen der vorliegenden Erfindung beruhen auf der Idee, bei der Fertigung eines Endoskops nicht lose Bündel von Lichtleitfasern einzusetzen, sondern eine Lichtleiteinrichtung mit einem gekrümmten starren Abschnitt am distalen Ende zu verwenden. Die derart vorgeformte Lichtleiteinrichtung kann außerhalb des Endoskops und damit räumlich und zeitlich beabstandet und logistisch unabhängig gefertigt und auf ihre Funktion geprüft werden. Als starre Einheit weist insbesondere das distale Ende der Lichtleiteinrichtung eine erhöhte mechanische Robustheit auf. Das Risiko einer Beschädigung der Lichtleiteinrichtung beim Einsetzen in den Schaft des zukünftigen Endoskops ist dadurch deutlich vermindert.

Eine Lichtleiteinrichtung für ein Endoskop, zum Leiten von Beleuchtungslicht zum distalen Ende des Endoskops, weist bereits vor dem Einsetzen eines Innenschafts in einen Außenschaft für das Endoskop einen gekrümmten starren Abschnitt mit einer vorbestimmten räumlichen Konfiguration auf.

Eine Lichtleiteinrichtung für ein Endoskop, zum Leiten von Beleuchtungslicht zum distalen Ende des Endoskops, umfasst einen gekrümmten starren Abschnitt mit einer vorbestimmten räumlichen Konfiguration, wobei der gekrümmte starre Abschnitt zur Anordnung an einem distalen Ende eines Endoskops vorgesehen ist, wobei der gekrümmte starre Abschnitt seine starre Eigenschaft zumindest entweder bereits vor einem Einsetzen der Lichtleiteinrichtung in ein Endoskop oder vor Ausbildung einer unmittelbaren oder mittelbaren mechanischen Verbindung der Lichtleiteinrichtung mit einem Innenschaft für ein Endoskop oder vor Ausbildung einer unmittelbaren oder mittelbaren mechanischen Verbindung der Lichtleiteinrichtung mit einem Außenschaft für ein Endoskop aufweist. Eine Krümmung der Lichtleiteinrichtung im Sinne der vorliegenden Erfindung ist eine Krümmung des für Beleuchtungslicht vorgesehenen Ausbreitungswegs innerhalb der Lichtleiteinrichtung. Der für Beleuchtungslicht vorgesehene Ausbreitungsweg wird insbesondere durch die glatte Linie, die durch die Flächenmittelpunkte der Querschnittsflächen der Lichtleiteinrichtung gebildet wird, repräsentiert, wobei die Querschnittsflächen insbesondere senkrecht zu der Linie sind. Beispielsweise liegen bei einer Gauß-Förmigen Verteilung des Lichtflusses von in die Lichtleiteinrichtung eingekoppeltem Beleuchtungslicht die Orte maximaler Lichtflussdichte bzw. maximaler Intensität in der Regel auf dieser Linie oder nahe bei dieser Linie.

Die Lichtleiteinrichtung weist den gekrümmten starren Abschnitt bereits vor dem vorübergehenden oder endgültigen Einsetzen des Innenschafts und insbesondere auch schon vor dem vorübergehenden Einsetzen eines Dummy-Innenschafts in den Außenschaft auf. Der gekrümmte starre Abschnitt der Lichtleiteinrichtung wird also insbesondere außerhalb des Außenschafts und insbesondere zeitlich und räumlich beabstandet von der Fertigung anderer Komponenten des Endoskops erzeugt.

Die zeitliche und räumliche und damit auch logistische Trennung von der Fertigung anderer Bestandteile des Endoskops kann die Fertigungskosten reduzieren. Ferner kann die Lichtleiteinrichtung vor dem Einsetzen getestet werden, wobei aufgrund der Starrheit des gekrümmten Abschnitts eine Beschädigung beim Einsetzen - beispielsweise verglichen mit dem Einsetzen eines flexiblen Bündels von Lichtleitfasern - geringer ist.

Der starre Abschnitt der Lichtleiteinrichtung kann die gesamte Lichtleiteinrichtung umfassen und sich somit von der Lichteintrittsfläche bis zur Lichtaustrittsfläche der Lichtleiteinrichtung erstrecken. Alternativ kann der starre Abschnitt lediglich einen Teil der Lichtleiteinrichtung umfassen, wobei der starre Abschnitt insbesondere an die Lichtaustrittsfläche angrenzt oder den Bereich der Lichtleiteinrichtung umfasst, in dem nach dem Einsetzen der Lichtleiteinrichtung in einen Außenschaft für ein Endoskop eine Lichtaustrittsfläche erzeugt wird. Der starre Abschnitt kann neben einem oder mehreren gekrümmten Bereichen einen oder mehrere gerade Bereiche umfassen.

Die vorbestimmte räumliche Konfiguration des gekrümmten starren Abschnitts kann neben einer oder mehreren Krümmungen mit einem oder mehreren festen oder kontinuierlich oder diskontinuierlich variierenden Krümmungsradien auch Übergänge zwischen unterschiedlichen Querschnittsflächen mit konstantem oder variierendem Flächeninhalt umfassen.

Die Lichtleiteinrichtung ist insbesondere für ein Endoskop mit einer feststehenden oder einstellbaren Blickrichtung, die nicht parallel zur Längsachse des Endoskops ist, ausgebildet. Insbesondere bei einem Winkel von mehr als 30 Grad oder 45 Grad zwischen der Blickrichtung und der Längsachse des Endoskops ergeben sich aus der erforderlichen entsprechenden Krümmung der Lichtleiteinrichtung die oben beschriebenen Probleme, die mit der erfindungsgemäßen Lichtleiteinrichtung teilweise oder vollständig gelöst werden können.

Der Innenschaft umfasst insbesondere den Beobachtungsstrahlengang mit einem Objektiv oder einer anderen Abbildungseinrichtung, einer Stablinsenoptik oder einem geordneten Bündel von Lichtleitfasern oder einem Bildsensor. Die Lichtleiteinrichtung ist insbesondere zur Anordnung in einem Zwischenraum zwischen dem Innenschaft und dem Außenschaft vorgesehen.

Die Lichtleiteinrichtung mit dem gekrümmten starren Abschnitt kann also als vollständig separates Bauteil oder als teilweise oder vollständig mit dem Innenschaft gefügtes Bauteil ausgebildet sein.

Eine Lichtleiteinrichtung, wie sie hier beschrieben ist, umfasst insbesondere eine Mehrzahl von Lichtleitfasern, die im starren Abschnitt der Lichtleiteinrichtung miteinander gefügt und gekrümmt sind.

Eine Lichtleitung ist alternativ beispielsweise in dünnen Stäben aus transparentem Material möglich. Die lichtleitende Eigenschaft kann jedoch teilweise oder vollständig verlorengehen, wenn das Verhältnis zwischen dem Krümmungsradius und dem Durchmesser des Stabs nicht groß genug ist. Bei kleinen Krümmungsradien können deshalb möglichst dünne Lichtleitfasern gegenüber lichtleitenden Stäben oder dicken Lichtleitfasern den Vorteil eines deutlich geringeren Verlusts an Beleuchtungslicht aufweisen. Alternativ weist die Lichtleiteinrichtung eine Schichtstruktur, ein Übergitter oder eine andere innere Struktur auf, die eine Lichtleitung entlang eines vorgesehenen gekrümmten Ausbreitungswegs unterstützt.

Die Lichtleitfasern sind insbesondere miteinander verschweißt oder bei einer Temperatur nahe der Schmelztemperatur oder der Glasübergangs- oder Erweichungstemperatur miteinander verpresst. Dies ermöglicht eine besonders robuste stoffschlüssige Verbindung. Beim Verschweißen oder beim heißen Verpressen der Lichtleitfasern können ferner Zwischenräume zwischen den Lichtleitfasern entfernt werden. Dabei werden insbesondere ursprünglich kreisförmige Querschnitte der einzelnen Lichtleitfasern hexoagonal oder anders polygonal. Dadurch kann der gekrümmte starre Abschnitt einen fluiddichten Teil einer Oberfläche eines Endoskops bilden, der auch beim Autoklavieren des Endoskops kein Eindringen von Feuchtigkeit zulässt. Alternativ können die Lichtleitfasern mit einem metallischen oder nicht metallischen Lot verlötet, verklebt oder vergossen sein, wobei der gekrümmte starre Abschnitt ebenfalls fluiddicht sein kann.

Bei einer Lichtleiteinrichtung, wie sie hier beschrieben ist, mit einer Mehrzahl von miteinander gefügten Lichtleitfasern können die einzelnen Lichtleitfasern jeweils einen Querschnitt mit in Lichtausbreitungsrichtung variierendem Flächeninhalt aufweisen.

Durch Variieren der Querschnitte der einzelnen Lichtleitfasern kann die Divergenz bzw. der Raumwinkelbereich, in den an den Lichtaustrittsflächen der Lichtleitfasern Beleuchtungslicht austritt, eingestellt werden. Damit kann insbesondere der Raumwinkelbereich, in den Beleuchtungslicht austritt, an die Größe des Sichtfelds angepasst werden.

Bei einer Lichtleiteinrichtung, wie sie hier beschrieben ist, sind insbesondere die Flächennormalen einer Lichteintrittsfläche und einer Lichtaustrittsfläche der Lichtleiteinrichtung nicht parallel.

Im Falle einer gekrümmten Lichteintrittsfläche oder einer gekrümmten Lichtaustrittsfläche ist mit der Flächennormalen die mittlere Flächennormale oder die Flächennormale in der Mitte der jeweiligen Fläche gemeint. Die Flächennormale der Lichteintrittsfläche kann parallel zur vorgesehenen Lichtausbreitungsrichtung in der Lichtleiteinrichtung unmittelbar lichtstromabwärts der Lichteintrittsfläche sein. Die Flächennormale der Lichtaustrittsfläche ist insbesondere parallel zur Lichtausbreitungsrichtung in der Lichtleiteinrichtung unmittelbar lichtstromaufwärts der Lichtaustrittsfläche. Die Flächennormale der Lichteintrittsfläche ist beispielsweise parallel oder im Wesentlichen parallel (Winkeldifferenz kleiner als 5 Grad oder kleiner als 10 Grad) zur Längsachse des Endoskops, für das die Lichtleiteinrichtung vorgesehen und ausgebildet ist. Beispielsweise ist bei einem Winkel von 90 Grad zwischen der Blickrichtung und der Längsachse eines Schafts eines Endoskops, für das die Lichtleiteinrichtung vorgesehen und ausgebildet ist, der Winkel zwischen den Flächennormalen der Lichteintrittsfläche und der Lichtaustrittsfläche der Lichtleiteinrichtung 90 Grad. Zusätzlich zu einer Lichtaustrittsfläche, deren Flächennormale nicht parallel zur Flächennormale einer Lichteintrittsfläche der Lichtleiteinrichtung ist, kann die Lichtleiteinrichtung eine oder mehrere Lichtaustrittsflächen aufweisen, deren Flächennormalen parallel zur Flächennormal der Lichteintrittsfläche sind.

Die obigen Ausführungen zu den Flächennormalen von Lichtein- und -austrittsflächen sind insbesondere, aber nicht ausschließlich auf Lichtleiteinrichtungen bezogen, bei denen die Lichtausbreitungsrichtung unmittelbar lichtstromabwärts einer Lichteintrittsfläche parallel zur Flächennormalen der Lichteintrittsfläche und die Lichtausbreitungsrichtung unmittelbar lichtstromaufwärts einer Lichtaustrittsfläche parallel zur Flächennormelen der Lichtaustrittsfläche ist. Alternativ kann die Lichtleiteinrichtung an der Lichteintrittsfläche und/oder an der Lichtaustrittsfläche je einen Schräganschliff aufweisen. Bei einem Schräganschliff ist in der Lichtleiteinrichtung die Lichtausbreitungsrichtung unmittelbar lichtstromabwärts einer Lichteintrittsfläche nicht parallel zur Flächennormalen der Lichteintrittsfläche bzw. die Lichtausbreitungsrichtung unmittelbar lichtstromaufwärts einer Lichtaustrittsfläche nicht parallel zur Flächennormalen der Lichtaustrittsfläche.

Eine Lichtleiteinrichtung, wie sie hier beschrieben ist, mit oder ohne Schräganschliff an einer Lichteintrittsfläche oder an einer Lichtaustrittsfläche kann für eine Lichtausbreitungsrichtung unmittelbar lichtstromabwärts der Lichteintrittsfläche und eine Lichtausbreitungsrichtung unmittelbar lichtstromaufwärts der Lichtaustrittsfläche ausgebildet sein, die nicht parallel sind.

Eine Lichtleiteinrichtung, wie sie hier beschrieben ist, weist insbesondere einen im gekrümmten starren Abschnitt entlang des vorgesehenen Ausbreitungswegs von Beleuchtungslicht variierenden Querschnitt auf.

Der Ausbreitungsweg des Beleuchtungslichts entspricht gegebenenfalls dem Verlauf der Lichtleitfasern in der Lichtleiteinrichtung. Der Querschnitt der Lichtleiteinrichtung kann im starren Abschnitt variieren, ohne dass gegebenenfalls die Querschnitte der Lichtleitfasern variieren. Insbesondere bei Lichtleitfasern mit konstantem Querschnitt kann der Flächeninhalt des Querschnitts der Lichtleiteinrichtung im starren Abschnitt entlang des vorgesehenen Ausbreitungswegs von Beleuchtungslicht konstant sein.

Mit einem variierenden Querschnitt kann der starre Abschnitt der Lichtleiteinrichtung an den zur Verfügung stehenden Bauraum angepasst werden, der insbesondere nahe dem distalen Ende eines Endoskop typischerweise in Längsrichtung des Schafts variiert.

Eine Lichtleiteinrichtung, wie sie hier beschrieben ist, kann bereits vor dem Einsetzen eines Innenschafts in einen Außenschaft für ein Endoskop entweder mit dem Innenschaft oder mit dem Außenschaft gefügt sein.

Wie bereits erwähnt, ist der Innenschaft typischerweise ausgebildet, um den Beleuchtungsstrahlengang zu umfassen bzw. zu enthalten. Der Außenschaft eines Endoskops ist typischerweise ausgebildet, um den Innenschaft mit Beleuchtungsstrahlengang und die Lichtleiteinrichtung zu umfassen. Die Lichtleiteinrichtung kann mit der äußeren Oberfläche des Innenschafts oder mit der inneren Oberfläche des Außenschafts verschweißt oder bei einer Temperatur im Bereich der Schmelztemperatur oder der Glasübergangs- oder Erweichungstemperatur des Materials der Lichtleiteinrichtung verpresst sein. Dadurch kann eine mechanisch besonders robuste Einheit aus der Lichtleiteinrichtung und dem Innenschaft oder aus der Lichtleiteinrichtung und dem Außenschaft gebildet werden, bevor der Innenschaft in den Außenschaft eingesetzt wird. Alternativ kann die Lichtleiteinrichtung mit dem Innenschaft oder mit dem Außenschaft mittels eines metallischen oder nichtmetallischen Lots verlötet, verklebt oder vergossen sein.

Nach dem Einsetzen des bereits mit der Lichtleiteinrichtung eine mechanische Einheit bildenden Innenschafts in den Außenschaft oder des Innenschafts in den bereits mit der Lichtleiteinrichtung eine mechanische Einheit bildenden Außenschaft können Innenschaft und Außenschaft miteinander durch Stoffschluss und/oder Kraftschluss und/oder Formschluss verbunden werden. Danach ist die bereits vor dem Einsetzen des Innenschafts in den Außenschaft mit einem der beiden eine mechanische Einheit bildende Lichtleiteinrichtung sowohl mit dem Innenschaft als auch mit dem Außenschaft jeweils unmittelbar oder mittelbar mechanisch verbunden.

Bei einer Lichtleiteinrichtung, wie sie hier beschrieben ist, kann die Lichteintrittsfläche der Lichtleiteinrichtung vorgesehen und ausgebildet sein, um am proximalen Ende eines Endoskops angeordnet zu werden.

Wenn die Lichtleiteinrichtung eine Mehrzahl von Lichtleitfasern umfasst, wird die Lichteintrittsfläche der Lichtleiteinrichtung durch die Lichteintrittsflächen der Lichtleitfasern gebildet. Die Lichteintrittsfläche der Lichtleiteinrichtung ist insbesondere ausgebildet, um am proximalen Ende eines Endoskops mit einer in das proximale Ende des Endoskops integrierten Lichtquelle oder über ein Lichtleitkabel mit einer externen Lichtquelle gekoppelt zu werden.

Die Lichtleiteinrichtung kann von der zur Anordnung am proximalen Ende eines Endoskops vorgesehenen und ausgebildeten Lichteintrittsfläche bis zur Anordnung am distalen Ende des Endoskops vorgesehenen Lichtaustrittsfläche starr ausgebildet sein. Alternativ kann die Lichtleiteinrichtung einen oder mehrere flexible Abschnitte aufweisen. Wenn die Lichtleiteinrichtung eine Mehrzahl von Lichtleitfasern umfasst, sind diese insbesondere nur in einem kurzen gekrümmten starren Abschnitt, der sich an die Lichtaustrittsfläche der Lichtleiteinrichtung anschließt, miteinander gefügt, so dass die Lichtleiteinrichtung lichtstromaufwärts des gekrümmten starren Abschnitts aufgrund der Flexibilität der einzelnen und nicht miteinander gefügten Lichtleitfasern flexibel ist.

Eine zumindest abschnittsweise flexible Ausbildung der Lichtleiteinrichtung kann das Entstehen von mechanischen Spannungen aufgrund unterschiedlicher thermischer Ausdehnungskoeffizienten beispielsweise beim Autoklavieren des fertigen Endoskops verhindern und damit die Lebensdauer eines mit der Lichtleiteinrichtung hergestellten Endoskops erhöhen.

Bei einer Lichtleiteinrichtung, wie sie hier beschrieben ist, kann die Lichteintrittsfläche vorgesehen und ausgebildet sein, um an einem anderen Ort als am proximalen Ende eines Endoskops angeordnet zu werden, insbesondere im Schaft des Endoskops.

Insbesondere ist die Lichteintrittsfläche vorgesehen und ausgebildet, um nahe dem distalen Ende eines Endoskops angeordnet zu werden, wobei die Lichtleiteinrichtung insbesondere vollständig starr ausgebildet ist bzw. der gekrümmte starre Abschnitt die gesamte Lichtleiteinrichtung umfasst. Die Lichteintrittsfläche der Lichtleiteinrichtung ist in diesem Fall vorgesehen, um unmittelbar mit einer Lichtquelle nahe dem distalen Ende des Endoskops oder mittels eines weiteren Lichtleiters mit einer Lichtquelle am proximalen Ende des Endoskops oder mittels eines weiteren Lichtleiters und eines vom Endoskop abnehmbaren Lichtleitkabels mit einer separaten Lichtquelle optisch gekoppelt zu werden. Die Lichteintrittsfläche der Lichtleiteinrichtung kann insbesondere mit einer Lichtaustrittsfläche des weiteren Lichtleiters verkittet bzw. mittels eines transparenten Materials stoffschlüssig verbunden werden.

Eine Lichtleiteinrichtung, wie sie hier beschrieben ist, weist insbesondere mehrere Lichtaustrittsflächen auf, die für einen Lichtaustritt in unterschiedliche Raumwinkelbereiche ausgebildet sind.

Insbesondere schließen die Flächennormalen von Lichtaustrittsflächen der Lichtleiteinrichtung Winkel ein, die mindestens 15 Grad oder mindestens 30 Grad betragen. Mit mehreren Lichtaustrittsflächen ist die Lichtleiteinrichtung in der Lage - abhängig von der Einkopplung von Beleuchtungslicht in eine oder mehrere Lichteintrittsflächen der Lichtleiteinrichtung - gleichzeitig oder alternativ Beleuchtungslicht in mehrere getrennte oder überlappende Raumwinkelbereiche abzustrahlen. Damit ist die Lichtleiteinrichtung insbesondere für ein Endoskop mit einstellbarer Blickrichtung geeignet.

Eine Lichtleiteinrichtung, wie sie hier beschrieben ist, umfasst insbesondere mehrere Bündel von Lichtleitfasern mit je einem starren Abschnitt.

Die Bündel von Lichtleitfasern können miteinander mechanisch starr verbunden sein und dabei insbesondere eine gemeinsame oder mehrere getrennte Lichteintrittsflächen und eine gemeinsame oder mehrere voneinander getrennte Lichtaustrittsflächen aufweisen. Insbesondere sind die starren Abschnitte, von denen mindestens einer abschnittsweise gekrümmt ist, miteinander gefügt. Alternativ sind die Bündel von Lichtleitfasern nicht mechanisch verbunden, aber in ihrer räumlichen Gestalt aufeinander abgestimmt. Dabei können die Bündel von Lichtleitfasern separat hergestellt sein.

Mehrere Bündel von Lichtleitfasern ermöglichen, insbesondere bei separaten Lichtaustrittsflächen, eine alternative oder gleichzeitige Abstrahlung von Beleuchtungslicht in unterschiedliche Raumwinkelbereiche.

Ein Endoskop umfasst eine Lichtleiteinrichtung, wie sie hier beschrieben ist.

Das Endoskop ist insbesondere für eine Blickrichtung ausgebildet, die nicht parallel zur Längsachse des Endoskops ist.

Beispielsweise ist das Endoskop für eine oder mehrere feste Blickrichtungen ausgebildet, die mit der Längsachse des Schafts des Endoskops Winkel von 30 Grad, 45 Grad, 60 Grad, 90 Grad einschließen. Alternativ ist das Endoskop beispielweise für ein kontinuierliches Spektrum von Blickrichtungen, die mit der Längsachse des Schafts des Endoskops Winkel in einem vorbestimmten Winkelintervall einschließen, ausgebildet, wobei das Winkelintervall beispielsweise von 0 Grad bis 120 Grad oder von 45 Grad bis 120 Grad reicht.

Bei einem Endoskop, wie es hier beschrieben ist, ist die Lichtleiteinrichtung entweder mit einem Außenschaft oder mit einem Innenschaft des Endoskops (10) gefügt.

Insbesondere ist die Lichtleiteinrichtung entweder mit dem Innenschaft oder mit dem Außenschaft nicht gefügt.

Bei einem Verfahren zum Herstellen einer Lichtleiteinrichtung für ein Endoskop zum Leiten von Beleuchtungslicht zum distalen Ende des Endoskops wird ein gekrümmter starrer Abschnitt der Lichtleiteinrichtung, in dem die Lichtleiteinrichtung eine vorbestimmte räumliche Konfiguration aufweist, erzeugt, wobei der gekrümmte starre Abschnitt zur Anordnung an einem distalen Ende eines Endoskops vorgesehen ist, wobei der Schritt des Erzeugens zumindest entweder vor einem Einsetzen der Lichtleiteinrichtung in ein Endoskop oder vor einem unmittelbaren oder mittelbaren mechanischen Verbinden der Lichtleiteinrichtung mit einem Innenschaft für ein Endoskop oder vor einem unmittelbaren oder mittelbaren mechanischen Verbinden der Lichtleiteinrichtung mit einem Außenschaft für ein Endoskop ausgeführt wird.

Bei einem Verfahren, wie es hier beschrieben ist, umfasst der Schritt des Erzeugens eines gekrümmten starren Abschnitts insbesondere ein Anordnen, ein Krümmen und ein Fügen von Lichtleitfasern in einem Abschnitt in einer vorbestimmten gekrümmten räumlichen Konfiguration, wobei der Abschnitt, in dem die Lichtleitfasern miteinander gefügt und gekrümmt sind, zur Anordnung an einem distalen Ende eines Endoskops vorgesehen ist.

Die Schritte des Anordnens, Krümmens und Fügens können in der angegebenen Reihenfolge oder in einer anderen Reihenfolge oder zumindest teilweise gleichzeitig ausgeführt werden. Die Lichtleitfasern werden insbesondere durch Verschweißen oder durch Pressen bei einer Temperatur im Bereich der Schmelztemperatur oder der Glasübergangs- oder Erweichungstemperatur des Materials der Lichtleitfasern gefügt. Alternativ können die Lichtleitfasern mittels eines metallischen oder nicht metallischen Lots verlötet, verklebt oder vergossen werden.

Bei einem Verfahren zum Herstellen einer Lichtleiteinrichtung, wie es hier beschrieben ist, wird insbesondere eine Lichteinrichtung, wie sie hier beschrieben ist, hergestellt.

Bei einem Verfahren zum Herstellen eines Endoskops wird eine Lichtleiteinrichtung, wie sie hier beschrieben ist, oder eine nach einem Verfahren, wie es hier beschrieben ist, hergestellte Lichtleiteinrichtung in einen Schaft für ein Endoskop eingesetzt.

Das Verfahren zum Herstellen eines Endoskops kann weitere Schritte umfassen, beispielsweise ein Herstellen einer Lichtleiteinrichtung nach den oben beschriebenen Verfahren.

### Kurzbeschreibung der Figuren

Nachfolgend werden Ausführungsformen anhand der beigefügten Figuren näher erläutert. Es zeigen:
- Figur 1: eine schematische Darstellung eines Endoskops;
- Figur 2: eine schematische Darstellung eines weiteren Endoskops;
- Figur 3: eine weitere schematische Darstellung einer Lichtleiteinrichtung aus Figur 2;
- Figur 4: eine schematische Darstellung eines ersten Querschnitts der Lichtleiteinrichtung aus Figur 3;
- Figur 5: eine schematische Darstellung eines zweiten Querschnitts der Lichtleiteinrichtung aus Figur 3;
- Figur 6: eine schematische Darstellung eines dritten Querschnitts der Lichtleiteinrichtung aus Figur 3;
- Figur 7: ein schematisches Flussdiagramm eines Verfahrens zum Herstellen eines Endoskops.

### Beschreibung der Ausführungsformen

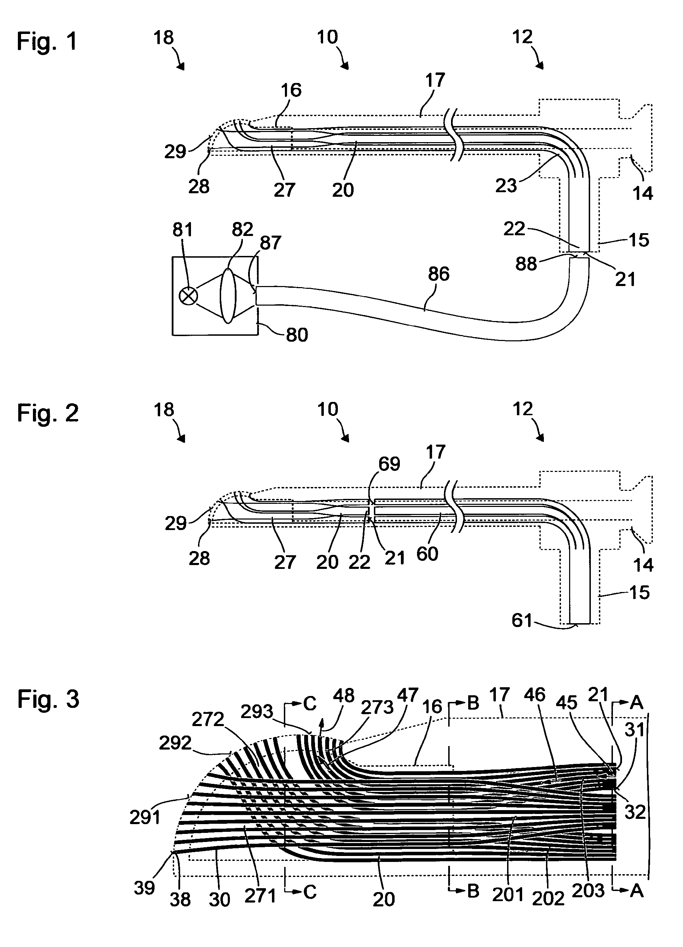

Figur 1 zeigt eine schematische Darstellung eines Endoskops 10 mit einer Lichtleiteinrichtung 20. Da die Lichtleiteinrichtung 20 und deren Merkmale im Fokus dieser Anmeldung stehen und außerhalb des Endoskops 10 hergestellt bzw. erzeugt werden können, ist das Endoskop 10 in gestrichelten Linien dargestellt.

Das Endoskop 10 weist ein proximales Ende 12 mit einem Okular 14 und einer Kupplung 15 für ein Lichtleitkabel und ein distales Ende 18 auf. Zwischen dem proximalen Ende 12 und dem distalen Ende 18 erstreckt sich ein Außenschaft 17, in dem die Lichtleiteinrichtung 20 und ein Innenschaft 16 mit einem hier nicht weiter beschriebenen Beobachtungsstrahlengang angeordnet sind. Der Innenschaft 16 erstreckt sich vom distalen Ende 18 bis zum proximalen Ende 12 des Endoskops und weist einen Querschnitt auf, der über einen großen Bereich konstant ist. Am distalen Ende 18 des Endoskops 10 weisen der Innenschaft 16 und der Außenschaft 17 jeweils nicht-konstante Querschnitte auf, die insbesondere durch Einrichtungen für eine einstellbare Blickrichtung des Endoskops 10 bedingt sind.

Eine Lichtquelleneinrichtung 80 mit einer Lichtquelle 81 und einer Abbildungseinrichtung 82 ist über ein Lichtleitkabel 86 mit der Kupplung 15 am proximalen Ende 12 des Endoskops 10 gekoppelt. Die Abbildungseinrichtung 82, insbesondere ein Objektiv oder eine oder mehrere Linsen oder Spiegel, bildet die Lichtquelle 81 auf eine Lichteintrittsfläche 87 des Lichtleitkabels 86 ab. Eine Lichtaustrittsfläche 88 des Lichtleitkabels 86 wird von mechanischen Kupplungseinrichtungen am Lichtleitkabel 86 und an der Kupplung 15 am Endoskop 10 gegenüber einer Lichteintrittsfläche 21 der Lichtleiteinrichtung 20 im Endoskop 10 gehalten. Die Kupplungseinrichtungen am Lichtleitkabel 86 und an der Kupplung 15 sind in Figur 1 nicht dargestellt. Die Lichtaustrittsfläche 88 des Lichtleitkabels 86 und die Lichteintrittsfläche 21 der Lichtleiteinrichtung 20 sind in Figur 1 beabstandet voneinander dargestellt. Tatsächlich können sie durch die genannten Kupplungseinrichtungen gegeneinander gedrückt bzw. gepresst werden.

Die Lichtleiteinrichtung 20 ist von ihrem proximalen Ende 22 an ihrer Lichteintrittsfläche 21 bis zu ihrem distalen Ende 28 mit mehreren Lichtaustrittsflächen 29 mehrfach gekrümmt und weist einen variierenden Querschnitt auf. In Figur 1 ist insbesondere erkennbar, dass die Lichtleiteinrichtung 20 lichtstromabwärts der Kupplung 15 in mehrere Stränge bzw. Bündel aufgespalten ist, die über größere Strecken parallel verlaufen und nahe dem distalen Ende 18 des Endoskops 10 sich teilweise kreuzen.

Die Lichtleiteinrichtung 20 kann einen flexiblen Bereich 23 aufweisen, der insbesondere eine Krümmung zwischen der Kupplung 15 und dem Außenschaft 17 des Endoskops 10 umfasst. Der flexible Bereich 23 kann sich bis nahe dem distalen Ende 18 des Endoskops 10 erstrecken, insbesondere näherungsweise in dem gesamten Bereich, in dem die Lichtleiteinrichtung 20 in Figur 1 gerade dargestellt ist. Alternativ kann die gesamte Lichtleiteinrichtung 20 starr ausgebildet sein.

Figur 2 zeigt eine schematische Darstellung eines weiteren Endoskops mit einer weiteren Lichtleiteinrichtung 20, die in einigen Merkmalen der oben anhand der Figur 1 dargestellten Lichtleiteinrichtung ähnelt. Die in Figur 2 dargestellte Lichtleiteinrichtung 20 ist kürzer als die oben anhand der Figur 1 dargestellte Lichtleiteinrichtung. Insbesondere sind das proximale Ende 22 und die Lichteintrittsfläche 21 der Lichtleiteinrichtung 20 nicht am proximalen Ende 12 des Endoskops 10 im Bereich der Kupplung 15, sondern im Außenschaft 17 nahe dem distalen Ende 18 des Endoskops 10 angeordnet.

Von der Kupplung 15 des Endoskops 10 bis zum proximalen Ende 22 der Lichtleiteinrichtung 20 erstreckt sich ein Lichtleiter 60. Eine Lichteintrittsfläche 61 des Lichtleiters 60 ist an der Kupplung 15 so angeordnet, dass sie mit einer Lichtaustrittsfläche eines Lichtleitkabels optisch gekoppelt werden kann. Eine Lichtaustrittsfläche 69 des Lichtleiters 60 ist der Lichteintrittsfläche 21 der Lichtleiteinrichtung 20 gegenüber angeordnet. In Figur 2 sind die Lichtaustrittsfläche 69 des Lichtleiters 60 und die Lichteintrittsfläche 21 der Lichtleiteinrichtung 20 beabstandet dargestellt. Tatsächlich können die Lichtaustrittsfläche 69 des Lichtleiters 60 und die Lichteintrittsfläche 21 der Lichtleiteinrichtung 20 für eine optimale Kopplung unmittelbar aneinander angrenzend angeordnet, mechanisch gegeneinander gepresst und/oder durch einen optischen Kitt oder ein anderes transparentes Material mit geeigneten optischen Eigenschaften mechanisch und optisch miteinander gekoppelt sein.

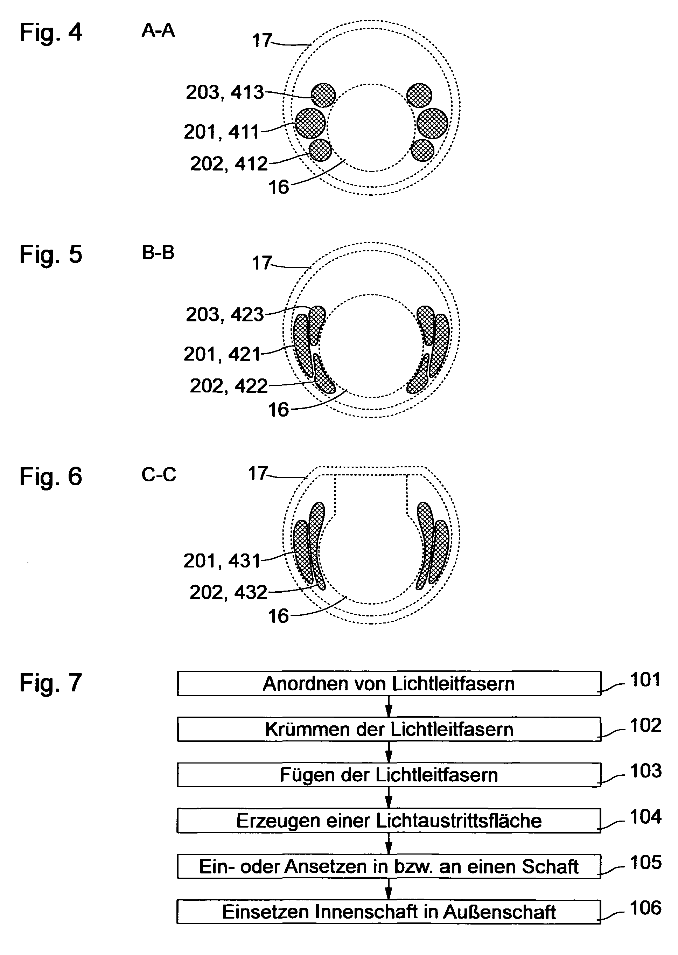

Figur 3 zeigt eine weitere schematische Darstellung der Lichtleiteinrichtung aus Figur 2. In der vergrößerten Darstellung der Figur 3 ist erkennbar, dass die Lichtleiteinrichtung 20 mehrere Bündel 201, 202, 203 von Lichtleitfasern 30 umfasst. In Figur 3 sind die Lichtleitfasern 30 innerhalb jedes Bündels 201, 202, 203 geordnet nebeneinander, im Wesentlichen parallel und mit gleichen Abständen dargestellt. Durch in Längsrichtung der Lichtleitfasern 30 variierende gegenseitige Abstände der Lichtleitfasern innerhalb eines Bündels 201, 202, 203 sind unterschiedliche Querschnitte der Bündel 201, 202, 203 angedeutet. Diese unterschiedlichen Querschnitte der Bündel 201, 202, 203 sind unten mit Bezug auf die Figuren 4 bis 6 näher beschrieben. Die schematische Darstellung in Figur 3 ist jedoch nicht so zu verstehen, dass die Bündel 201, 202, 203 quasi-eindimensionale Querschnitte aufweisen, innerhalb derer die Lichtleitfasern 30 ausschließlich bandförmig bzw. nebeneinander angeordnet sind.

Innerhalb einer Fläche, insbesondere innerhalb einer Ebene angeordnete Lichteintrittsflächen 31 der Lichtleitfasern 30 bilden die Lichteintrittsfläche 21 der Lichtleiteinrichtung 20. Die Lichteintrittsfläche 21 umfasst eine der Anzahl der Bündel 201, 202, 203 entsprechende Mehrzahl von nicht zusammenhängenden Teilflächen. Alternativ können die Bündel 201, 202, 203 im Bereich der Lichteintrittsfläche 21 abweichend von der Darstellung in Figur 3 zusammengefasst sein, so dass die Lichteintrittsflächen 31 der Lichtleitfasern 30 aller Bündel 201, 202, 203 eine einzige zusammenhängende Lichteintrittsfläche 21 bilden.

Die Lichtaustrittsflächen 39 an den distalen Enden 38 der Lichtleitfasern 30 eines Bündels 201, 202, 203 bilden die Lichtaustrittsfläche 291, 292, 293 des entsprechenden Bündels 201, 202, 203. Bei dem in Figur 3 dargestellten Beispiel weist ein erstes Bündel 201 nahe seiner Lichtaustrittsfläche 291 einen allenfalls schwach gekrümmten Bereich 271 auf. Ein zweites Bündel 202 und ein drittes Bündel 203 weisen lichtstromaufwärts ihrer Lichtaustrittsflächen 292, 293 stark gekrümmte starre Bereiche 272, 273 auf. Das zweite Bündel 202 und das dritte Bündel 203 weisen ferner lichtstromabwärts der Lichteintrittsfläche 21 schwach gekrümmte Bereiche auf. Der Krümmung des zweiten Bündels 202 und des dritten Bündels 203 entspricht eine entsprechende Krümmung der Lichtleitfasern 30 dieser Bündel.

Die vorgesehenen Ausbreitungswege von Beleuchtungslicht innerhalb der Lichtleiteinrichtung 20, insbesondere innerhalb der einzelnen Bündel 201, 202, 203 entsprechen dem Verlauf bzw. der Richtung der einzelnen Lichtleitfasern 30 innerhalb der Bündel 201, 202, 203. Am dritten Bündel 203 sind beispielhaft die Flächennormale 45 der Lichteintrittsfläche 21, eine Lichtausbreitungsrichtung 46 lichtstromabwärts der Lichteintrittsfläche 21, eine weitere Lichtausbreitungsrichtung 47 im stark gekrümmten starren Bereich 273 und eine mittlere Flächennormale der Lichtaustrittsfläche 293 des Bündels 203 durch Pfeile dargestellt. Die Flächennormalen 45, 48 der Lichteintrittsfläche 21 bzw. der Lichtaustrittsfläche 293 entsprechen bei dem dargestellten Beispiel im Wesentlichen den mittleren vorgesehenen Lichtausbreitungsrichtungen innerhalb des Bündels 203 unmittelbar lichtstromabwärts der Lichteintrittsfläche 21 bzw. unmittelbar lichtstromaufwärts der Lichtaustrittsfläche 293.

Die Lichtausbreitungsrichtung ist bei dem in Figur 3 dargestellten Beispiel zunächst im Wesentlichen parallel zur Längsachse des in Figur 3 in gestrichelten Linien angedeuteten Außenschafts 17 eines Endoskops, für das die Lichtleiteinrichtung 20 vorgesehen ist (Pfeil 45). Lichtstromabwärts der Lichteintrittsfläche 21 variiert die Lichtausbreitungsrichtung zunächst leicht (Pfeil 46), wobei das dritte Bündel 203 teilweise das erste Bündel 201 kreuzt. Weiter lichtstromabwärts ist die Lichtausbreitungsrichtung über eine kurze Distanz näherungsweise konstant, die Lichtleitfasern verlaufen im Wesentlichen parallel. Weiter lichtstromabwärts variiert die Lichtausbreitungsrichtung bis zur Lichtaustrittsfläche 293 um mehr als 90 Grad (Pfeile 47, 48).

Durch ein Verschweißen, Verschmelzen, Verlöten, Verkleben oder Vergießen der einzelnen Lichtleitfasern 30 sind die einzelnen Bündel 201, 202, 203 der Lichtleiteinrichtung 20 starr, und zwar insbesondere von der Lichteintrittsfläche 21 bis zu den Lichtaustrittsflächen 291, 292, 293. Die Bündel 201, 202, 203 können untereinander mechanisch starr verbunden oder als separate Bauteile ausgebildet sein.

Die Figuren 4 bis 6 zeigen schematische Darstellungen von Schnitten entlang den in Figur 3 angedeuteten Schnittebenen A-A, B-B bzw. C-C durch die Lichtleiteinrichtung 20 aus den Figuren 2 und 3. Die Schnittebenen A-A, B-B und C-C sind jeweils senkrecht zu den Zeichenebenen der Figuren 2 und 3. In gestrichelten Linien sind die Konturen des Innenschafts 16 und des Außenschafts 17 angedeutet. Während in Figur 3 nur drei Bündel 201, 202, 203 von Lichtleitfasern dargestellt sind, sind in den Figuren 4 bis 6 jeweils die Querschnitte von sechs Bündeln erkennbar, die paarweise symmetrisch zueinander an zwei gegenüberliegenden Seiten des Innenschafts 16 angeordnet sind. Jeweils drei an einer Seite des Innenschafts benachbart zueinander angeordnete Bündel 201, 202, 203 bilden eine Lichtleiteinrichtung 20. Alternativ bilden alle sechs Bündel zusammen eine Lichtleiteinrichtung 20. Nachfolgend wird aufgrund der Symmetrie nur auf drei an einer Seite des Innenschafts 16 benachbart zueinander angeordnete Bündel 201, 202, 203 Bezug genommen.

Alternativ können zwei an gegenüberliegenden Seiten des Innenschafts 16 angeordnete Lichtleiteinrichtungen abweichend von der Darstellung in den Figuren 4 bis 6 asymmetrisch zu einander ausgebildet sein. Beispielsweise weisen eine erste Lichtleiteinrichtung an einer Seite des Innenschafts Bündel und Lichtaustrittsflächen zum Abstrahlen von Beleuchtungslicht unter Winkeln von 0 Grad, 48 Grad und 96 Grad und eine zweite Lichtleiteinrichtung an einer gegenüberliegenden Seite des Innenschafts Bündel und Lichtaustrittsflächen zum Abstrahlen von Beleuchtungslicht unter Winkeln von 24 Grad, 72 Grad und 120 Grad auf.

Alternativ zu der Darstellung in den Figuren 4 bis 6 kann nur an einer Seite des Innenschafts eine Lichtleiteinrichtung angeordnet sein, die ein, zwei, drei oder mehr Bündel von Lichtleitfasern umfassen kann.

In der in Figur 4 dargestellten Ebene A-A weist jedes Bündel 201, 202, 203 (vgl. Figur 3) einen im Wesentlichen kreisförmigen Querschnitt 411 bzw. 412 bzw. 413 auf. Alternativ zu und abweichend von der Darstellung in Figur 4 können alle Bündel 201, 202, 203 in der Ebene A-A zu einem Bündel mit einem einzigen zusammenhängenden Querschnitt zusammengefasst sein. In der in Figur 5 dargestellten Ebene B-B weist jedes Bündel 201, 202, 203 einen abgeflachten und länglichen Querschnitt 421 bzw. 422 bzw. 423 auf. Dadurch finden die Bündel 201, 202, 203 trotz des im Bereich der Ebene B-B vergrößerten Querschnitts des Innenschafts 16 zu dessen Seiten Platz. Die Ebene C-C schneidet das dritte Bündel 203 nicht. Der Querschnitt 432 des im Bereich der Ebene C-C stark gekrümmten zweiten Bündels 202 ist besonders länglich auseinandergezogen, damit das gesamte zweite Bündel 202 durch den schmalen, zwischen dem Innenschaft 16 und dem ersten Bündel 201 zur Verfügung stehenden Raum hindurch passt. Im Vergleich der Figuren 3 und 6 ist erkennbar, dass die Lichtausbreitungsrichtung und die Längsachsen der Lichtleitfasern 30 des zweiten Bündels 202 im Bereich der Ebene C-C nicht senkrecht zur Ebene C-C sind.

Figur 7 zeigt ein schematisches Flussdiagramm eines Verfahrens zum Herstellen eines Endoskops. Obwohl das Verfahren auch zur Herstellung eines Endoskops mit einer Lichtleiteinrichtung, deren Merkmale von den oben anhand der Figuren 1 bis 6 dargestellten Merkmalen abweichen, geeignet ist, werden nachfolgend beispielhaft Bezugszeichen aus den Figuren 1 bis 6 verwendet, um die Verständlichkeit der Beschreibung zu verbessern.

Bei einem ersten Schritt 101 werden Lichtleitfasern 30 in einem oder mehreren Bündeln 201, 202, 203 angeordnet. Bei einem zweiten Schritt 102 werden die Lichtleitfasern 30 in eine vorbestimmte räumliche Konfiguration gekrümmt, wie sie beispielsweise in den Figuren 3 bis 6 erkennbar ist. Bei einem dritten Schritt 103 werden die Lichtleitfasern 30 miteinander gefügt, insbesondere bei einer Temperatur nahe der Schmelztemperatur oder der Glasübergangs- oder Erweichungstemperatur des Materials der Lichtleitfasern 30 miteinander verpresst, verschweißt, mittels eines metallischen oder nicht metallischen Lots verlötet, verklebt oder vergossen.

Die Lichtleitfasern 30 werden insbesondere wie in Figur 7 dargestellt, zunächst angeordnet, dann gekrümmt und dann gefügt. Die Reihenfolge dieser Schritte ist jedoch teilweise vertauschbar. Ferner können die Schritte teilweise gleichzeitig ausgeführt werden. Insbesondere können die Lichtleitfasern beim Anordnen in einer entsprechenden Negativform gekrümmt werden. Ferner können die Lichtleitfasern während des Fügens gekrümmt werden, also beispielsweise zunächst in einem oder mehreren Bündeln 201, 202, 203 angeordnet, dann auf eine Temperatur nahe der Schmelztemperatur oder der Glasübergangs- oder Erweichungstemperatur erwärmt, in die vorbestimmte räumliche Konfiguration gebracht und in dieser gepresst und so verschmolzen oder verschweißt werden.

Die Schritte des Anordnens 101, Krümmens 102 und Fügens 103 können für jedes Bündel 201, 202, 203 einer Lichtleiteinrichtung einzeln oder für mehrere oder alle Bündel 201, 202, 203 gleichzeitig ausgeführt werden. Dabei oder danach können die Bündel 201, 202, 203 aneinandergefügt werden. Mit den Schritten des Anordnens 101, des Krümmens 102 und des Fügens 103 wird ein gekrümmter starrer Abschnitt 272, 273 einer Lichtleiteinrichtung 20 erzeugt.

Bei einem vierten Schritt 104 können eine oder mehrere Lichtaustrittsflächen 29, 291, 292, 293 erzeugt werden, beispielsweise durch Schleifen und Polieren des oder der distalen Enden.

Die Schritte des Anordnens 101, des Krümmens 102 und des Fügens 103 und optional der Schritt des Erzeugens 204 einer Lichtaustrittsfläche 104 bilden zusammen ein Verfahren zum Herstellen einer Lichtleiteinrichtung 20, das räumlich und zeitlich beabstandet und logistisch unabhängig von anderen Verfahrensschritten zum Herstellen eines Endoskops 10 ausgeführt werden kann.

Bei einem fünften Schritt 105 wird die bei den vorangehenden Schritten hergestellte Lichtleiteinrichtung in einen Außenschaft 17 eingesetzt oder an einen Innenschaft 16 angesetzt, insbesondere mit dem Außenschaft 17 oder dem Innenschaft 16 gefügt. Ein Fügen der Lichtleiteinrichtung 20 mit einem Innenschaft 16 oder mit einem Außenschaft 17 kann ferner gleichzeitig mit dem Fügen 103 der Lichtleitfasern miteinander erfolgen. Bei einem sechsten Schritt 106 wird der Innenschaft 16 in den Außenschaft 17 eingesetzt. Dabei kann abhängig von der Ausführung des fünften Schritts 105 der Innenschaft 16 mit der Lichtleiteinrichtung 20 in den Außenschaft 17 eingesetzt oder der Innenschaft 16 in den mit der Lichtleiteinrichtung 20 versehenen Außenschaft 17 eingesetzt werden.

### Bezugszeichen

- 10: Endoskop
- 12: proximales Ende des Endoskops 10
- 14: Okular
- 15: Kupplung für Lichtleitkabel am proximalen Ende 12 des Endoskops 10
- 16: Innenschaft (mit Beobachtungsstrahlengang) des Endoskops 10
- 17: Außenschaft des Endoskops 10
- 18: distales Ende des Endoskops 10
- 20: Lichtleiteinrichtung
- 201: erstes Bündel von Lichtleitfasern 30
- 202: zweites Bündel von Lichtleitfasern 30
- 203: drittes Bündel von Lichtleitfasern 30
- 21: Lichteintrittsfläche der Lichtleiteinrichtung 20
- 22: proximales Ende der Lichtleiteinrichtung 20
- 23: flexibler Bereich der Lichtleiteinrichtung 20
- 27: gekrümmter starrer Abschnitt der Lichtleiteinrichtung 20
- 271: gekrümmter starrer Abschnitt des ersten Bündels 201 von Lichtleitfasern 30
- 272: gekrümmter starrer Abschnitt des zweiten Bündels 202 von Lichtleitfasern 30
- 273: gekrümmter starrer Abschnitt des dritten Bündels 203 von Lichtleitfasern 30
- 28: distales Ende der Lichtleiteinrichtung 20
- 29: Lichtaustrittsfläche der Lichtleiteinrichtung 20
- 291: Lichtaustrittsfläche des ersten Bündels 201 von Lichtleitfasern 30
- 292: Lichtaustrittsfläche des zweiten Bündels 202 von Lichtleitfasern 30
- 293: Lichtaustrittsfläche des dritten Bündels 203 von Lichtleitfasern 30
- 30: Lichtleitfaser
- 31: Lichteintrittsfläche der Lichtleitfaser 30
- 32: proximales Ende der Lichtleitfaser 30
- 38: distales Ende der Lichtleitfaser 30
- 39: Lichtaustrittsfläche der Lichtleitfaser 30
- 411: erster Querschnitt eines ersten Bündels 201 von Lichtleitfasern 30
- 412: erster Querschnitt eines zweiten Bündels 202 von Lichtleitfasern 30
- 413: erster Querschnitt eines dritten Bündels 203 von Lichtleitfasern 30
- 421: zweiter Querschnitt eines ersten Bündels 201 von Lichtleitfasern 30
- 422: zweiter Querschnitt eines zweiten Bündels 202 von Lichtleitfasern 30
- 423: zweiter Querschnitt eines dritten Bündels 203 von Lichtleitfasern 30
- 431: dritter Querschnitt eines ersten Bündels 201 von Lichtleitfasern 30
- 432: dritter Querschnitt eines zweiten Bündels 202 von Lichtleitfasern 30
- 45: Flächennormale der Lichteintrittsfläche des dritten Bündels 203
- 46: Lichtausbreitungsrichtung im dritten Bündel 203
- 47: Lichtausbreitungsrichtung im dritten Bündel 203
- 48: Flächennormale der Lichtaustrittsfläche 293 des dritten Bündels 203
- 60: Lichtleiter
- 61: Lichteintrittsfläche des Lichtleiters 60
- 69: Lichtaustrittsfläche des Lichtleiters 60
- 80: Lichtquelleneinrichtung
- 81: Lichtquelle der Lichtquelleneinrichtung
- 82: Abbildungseinrichtung der Lichtquelleneinrichtung
- 86: Lichtleitkabel
- 87: Lichteintrittsfläche des Lichtleitkabels 86
- 88: Lichtaustrittsfläche des Lichtleitkabels 86
- 101: erster Schritt (Anordnen von Lichtleitfasern)
- 102: zweiter Schritt (Krümmen der Lichtleitfasern)
- 103: dritter Schritt (Fügen der Lichtleitfasern)
- 104: vierter Schritt (Erzeugen einer Lichtaustrittsfläche)
- 105: fünfter Schritt (Einsetzen in Außenschaft bzw. Ansetzen an Innenschaft)
- 106: sechster Schritt (Einsetzen Innenschaft in Außenschaft)

## Patentansprüche

1. Lichtleiteinrichtung (20) für ein Endoskop (10), zum Leiten von Beleuchtungslicht zum distalen Ende (18) des Endoskops (10), mit:
einem gekrümmten starren Abschnitt (272, 273) mit einer vorbestimmten räumlichen Konfiguration,
wobei der gekrümmte starre Abschnitt (272, 273) zur Anordnung an einem distalen Ende (18) eines Endoskops (10) vorgesehen ist,
wobei der gekrümmte starre Abschnitt (272, 273) seine starre Eigenschaft zumindest entweder bereits vor einem Einsetzen der Lichtleiteinrichtung (20) in ein Endoskop (10) oder vor Ausbildung einer unmittelbaren oder mittelbaren mechanischen Verbindung der Lichtleiteinrichtung (20) mit einem Innenschaft (16) für ein Endoskop (10) oder vor Ausbildung einer unmittelbaren oder mittelbaren mechanischen Verbindung der Lichtleiteinrichtung (20) mit einem Außenschaft (17) für ein Endoskop (10) aufweist.

2. Lichtleiteinrichtung (20) nach einem der vorangehenden Ansprüche, wobei die Lichtleiteinrichtung (20) eine Mehrzahl von Lichtleitfasern (30) umfasst, die im gekrümmten starren Abschnitt (272, 273) der Lichtleiteinrichtung (20) miteinander gefügt und gekrümmt sind.

3. Lichtleiteinrichtung (20) nach dem vorangehenden Anspruch, wobei die einzelnen Lichtleitfasern (30) jeweils einen Querschnitt mit in Lichtausbreitungsrichtung (46, 47) variierendem Flächeninhalt aufweisen.

4. Lichtleiteinrichtung (20) nach einem der vorangehenden Ansprüche, bei der die Lichtleiteinrichtung (20) einen im gekrümmten starren Abschnitt (272, 273) entlang des vorgesehenen Ausbreitungswegs von Beleuchtungslicht variierenden Querschnitt (411, 412, 413, 421, 422, 423, 431, 432) aufweist.

5. Lichtleiteinrichtung (20) nach einem der vorangehenden Ansprüche, wobei die Lichtleiteinrichtung (20) bereits vor dem Einsetzen eines Innenschafts (16) in einen Außenschaft (17) für ein Endoskop entweder mit dem Innenschaft (16) oder mit dem Außenschaft (17) gefügt ist.

6. Lichtleiteinrichtung (20) nach einem der vorangehenden Ansprüche, wobei die Lichteintrittsfläche (21) der Lichtleiteinrichtung (20) vorgesehen und ausgebildet ist, um am proximalen Ende (12) eines Endoskops (10) angeordnet zu werden.

7. Lichtleiteinrichtung (20) nach einem der vorangehenden Ansprüche, wobei die Lichtleiteinrichtung (20) mehrere Lichtaustrittsflächen (29) aufweist, die für einen Lichtaustritt in unterschiedliche Raumwinkelbereiche ausgebildet sind.

8. Lichtleiteinrichtung (20) nach einem der vorangehenden Ansprüche, wobei die Lichtleiteinrichtung (20) mehrere Bündel (201, 202, 203) von Lichtleitfasern (30) mit je einem starren Abschnitt (271, 272, 273) umfasst.

9. Endoskop (10) mit einer Lichtleiteinrichtung (20) nach einem der vorangehenden Ansprüche.

10. Endoskop (10) nach dem vorangehenden Anspruch, wobei das Endoskop (10) für eine Blickrichtung ausgebildet ist, die nicht parallel zur Längsachse des Endoskops (10) ist.

11. Endoskop (10) nach Anspruch 9 oder 10, bei dem die Lichtleiteinrichtung entweder mit einem Außenschaft (17) oder mit einem Innenschaft (16) des Endoskops (10) gefügt ist.

12. Verfahren zum Herstellen einer Lichtleiteinrichtung (20) für ein Endoskop (10) zum Leiten von Beleuchtungslicht zum distalen Ende (18) des Endoskops (10), mit folgendem Schritt:
Erzeugen (101, 102, 103) eines gekrümmten starren Abschnitts (272, 273) der Lichtleiteinrichtung (20), in dem die Lichtleiteinrichtung (20) eine vorbestimmte räumliche Konfiguration aufweist,
wobei der gekrümmte starre Abschnitt (272, 273) zur Anordnung an einem distalen Ende eines Endoskops (10) vorgesehen ist, wobei der Schritt des Erzeugens (101, 102, 103) zumindest entweder vor einem Einsetzen der Lichtleiteinrichtung (20) in ein Endoskop (10) oder vor einem unmittelbaren oder mittelbaren mechanischen Verbinden der Lichtleiteinrichtung (20) mit einem Innenschaft (16) für ein Endoskop (10) oder vor einem unmittelbaren oder mittelbaren mechanischen Verbinden der Lichtleiteinrichtung (20) mit einem Außenschaft (17) für ein Endoskop (10) ausgeführt wird.

13. Verfahren nach dem vorangehenden Anspruch, bei dem der Schritt des Erzeugens folgende Schritte umfasst:
Anordnen (101) von Lichtleitfasern (30);
Krümmen (102) der Lichtleitfasern (30);
Fügen (103) der Lichtleitfasern (30) in einem Abschnitt (272_{;} 273) in einer vorbestimmten gekrümmten räumlichen Konfiguration,
wobei der Abschnitt (272, 273), in dem die Lichtleitfasern (30) miteinander gefügt und gekrümmt sind, zur Anordnung an einem distalen Ende (18) eines Endoskops (10) vorgesehen ist.

14. Verfahren nach Anspruch 12 oder 13, bei dem eine Lichtleiteinrichtung (20) nach einem der Ansprüche 1 bis 8 hergestellt wird.

15. Verfahren zum Herstellen eines Endoskops (10), mit folgendem Schritt:
Einsetzen (105) einer nach einem der Ansprüche 12 bis 14 hergestellten Lichtleiteinrichtung (20) in einen Außenschaft (17).
